# EUROPEAN PATENT APPLICATION

(11) **EP 4 822 013 A1**
(43) Date of publication of application: **14.10.2026**
(21) Application number: 25170236.1
(22) Date of filing: 12.04.2025
(51) Int. Cl.: C12N 1/14, C12P 19/40

(54) **A METHOD OF CULTURING CORDYCEPS FUNGI WITH A HIGH CONTENT OF CORDYCEPIN**

(71) Applicant: UAB Longevity Lab, 52159 Kaunas (LT)
(72) Inventor: Paskevicius, Sarunas, Kaunas (LT); Kazanaviciute, Vaiva, Kaunas (LT); Misiunas, Audrius, Kaunas (LT); Paule, Erika, Kaunas (LT)
(74) Representative: AAA Law

(57) **Abstract**

The present invention discloses a method for culturing *Cordyceps* fungi producing cordycepin, wherein the method comprises at least the steps: a) obtaining a fungi inoculum, b) optionally, inoculating the fungi inoculum in a first liquid growth medium, c) inoculating fungi inoculum into a second liquid growth medium, d) culturing under culturing conditions, wherein: (i) before or during step d) the second liquid growth medium is enriched with a source of oleic acid, preferably the source of oleic acid is insect material; and/or (ii) culturing conditions include a pulsed hyperoxygenation phase; and/or (iii) the *Cordyceps* species is a variant of *Cordyceps* species that has reduced capability to synthesize saturated fatty acids. The method of the present invention provides high concentrations of cordycepin and increased *Cordyceps* fungi biomass yield.

## Description

### FIELD OF INVENTION

The present invention relates to a culturing of *Cordyceps* fungi, in particular, to culturing *Cordyceps* fungi to obtain increased amounts of cordycepin.

### BACKGROUND OF INVENTION

The cultivation of medicinal fungi, such as *Cordyceps* species, has garnered significant interest due to their therapeutic properties, including immunomodulatory and anticancer effects (Thepmalee et al., 2024), cordycepin production being one of the interests. In particular, *Cordyceps militaris* is presently known to produce the most cordycepin among *Cordyceps* species. Traditional cultivation methods often face challenges such as prolonged growth cycles, inconsistent yields, and substantial energy consumption (Chang et al., 2024). These limitations hinder large-scale production and the efficient extraction of bioactive compounds.

Current methods for cultivating medicinal fungi primarily rely on three approaches: solid substrate cultivation, submerged fermentation process and liquid fermentation process. Solid substrate cultivation is a traditional technique that involves growing fungi on solid organic matter such as sawdust or grain. While well-established, it suffers from slow growth rates, inconsistency in yield, and significant dependence on environmental conditions. Variability in temperature, humidity, and nutrient availability results in heterogeneity, leading to fluctuating bioactive compound concentrations. Submerged fermentation method involves the cultivation of fungi in a liquid nutrient medium under continuous aeration and agitation. Unlike traditional liquid fermentation, submerged fermentation provides a highly controlled environment that optimizes fungal growth and metabolite production. However, challenges include maintaining the proper oxygen transfer rate, managing foam formation, and ensuring uniform nutrient distribution. Additionally, while submerged fermentation allows for higher biomass yields, achieving high concentrations of bioactive compounds remains difficult due to metabolic limitations and the need for optimized precursor feeding strategies (Amna et al., 2012). Liquid fermentation process - this method offers better control over environmental parameters and accelerates mycelial growth. However, it often fails to ensure high concentrations of bioactive compounds due to dilution effects and suboptimal metabolic conditions. Studies have indicated that bioactive compound yields can vary significantly depending on fermentation conditions.

One of the key challenges with the traditional methods is the inefficiency in extracting and producing bioactive compounds. The heterogeneity of conventional cultivation techniques does not guarantee consistent concentrations of medically relevant compounds such as cordycepin and polysaccharides. This limitation hinders standardization and scalability in pharmaceutical and nutraceutical applications.

Certain attempts have been made to grow *Cordyceps* by using insects with high fat content as substrates, i.e. where insects were used as solid substrates (KR20240015183A; Turk et al., 2022), however, the study was more focused on substrate selection rather than comprehensive cultivation parameters, with limited attention to scaling considerations for industrial production.

There is thus a need for improved *Cordyceps* cultivation methods and additives, which would result in increased cordycepin levels, overall higher *Cordyceps* biomass yield, and which could be easily scaled-up. The presently provided invention addresses various aspects of this need.

### SUMMARY OF INVENTION

The present inventors have identified that increased cordycepin amounts during cultivation of *Cordyceps* species, as well as higher yield *Cordyceps* mycelium biomass, can be achieved by cultivating *Cordyceps* species in a liquid medium enriched with insect biomass additives, by adding a two-step hyperoxygenation and/or by using variants of *Cordyceps* species that has reduced capability to synthesize saturated fatty acids.

In particular, the present invention discloses a method for culturing *Cordyceps* fungi producing cordycepin, comprising the steps:
a) Obtaining a fungi inoculum,
b) Optionally, inoculating the fungi inoculum in a first liquid growth medium,
c) Inoculating fungi inoculum into a second liquid growth medium,
d) culturing under culturing conditions,
wherein:
(i) before or during step d) the second liquid growth medium is enriched with a source of oleic acid, and/or
(ii) culturing conditions include include a pulsed hyperoxygenation phase, and/or
(iii) the *Cordyceps* species is a variant of *Cordyceps* species that has reduced capability to synthesize saturated fatty acids.

In some embodiments, the *Cordyceps* species is selected from *Cordyceps militaris*, *Cordyceps sinensis* (*Ophiocordyceps sinensis*), *Cordyceps cardinalis, Cordyceps cicadae, Cordyceps kyushuensis* and *Cordyceps bassiana,* preferably wherein the *Cordyceps* species is *Cordyceps militaris.*

In some embodiments, the fungi inoculum is obtained by inoculating the fungi mycelium or spores on a solid medium and incubating.

In some embodiments, the first liquid growth medium is the same as the second growth medium.

In some embodiments, the source of oleic acid is insect-derived material.

In some embodiments, the insect material is selected from the group consisting of insect biomass, insect biomass powder, insect extracts and insect homogenates.

In some embodiments, the insect material is selected from insects comprising 5-65 % (w/w) oleic acid content of total fatty acids, preferably, 20-45% (w/w) oleic acid content.

In some embodiments, the insect material is from insect family selected from the group consisting of *Tenebrionidae, Gryllidae, Acrididae, Acrididae, Bombycidae, Saturniidae, Formicidae, Scarabaeidae, Pyrrhocoridae, Carabidae, Lucanidae* insect families or combination thereof.

In some embodiments, the insect material is from insect genus selected from *Tenebrio, Acheta, Schistocerca, Chorthippus, Bombyx, Cricula, Formica, Protaetia, Dysdercus, Antheraea, Calosoma, Dorcus,* or combination thereof.

In some embodiments, the insect material is from insect selected from *Tenebrio molitor, Acheta domesticus, Schistocerca gregaria, Chorthippus brunneus, Bombyx mori, Cricula trifenestrata, Formica rufa, Protaetia brevitarsis, Dysdercus koenigii, Antheraea pernyi, Calosoma scrutator, Dorcus hopei,* or combination thereof.

In some embodiments, the variant of *Cordyceps* species that has reduced capability to synthesize saturated fatty acids is a Δfas1 mutant. In some embodiments, the Δfas1 mutant is a *Cordyceps militaris* Δfas1 mutant.

In some embodiments, when the method comprises culturing conditions that include the pulsed hyperoxygenation phase, the pulsed hyperoxygenation phase comprises a step comprising at least four first hyperoxygenation cycles of varying dissolved oxygen levels, wherein each first hyperoxygenation cycle comprises:
(i) maintaining dissolved oxygen at 45%-55%, preferably at 50%, of air saturation for 4-5 hours, preferably for 4 hours, followed by
(ii) maintaining dissolved oxygen at 15%-20%, preferably at 20%, of air saturation for 2-3 hours, preferably for 2 hours.

In further embodiments, the pulsed hyperoxygenation phase further comprises a step comprising at least four second hyperoxygenation cycles of varying dissolved oxygen levels, wherein each second hyperoxygenation cycle comprises:
(i) maintaining dissolved oxygen at 65%-75%, preferably at 70%, of air saturation for 4-5 hours, preferably for 4 hours, followed by
(ii) maintaining dissolved oxygen at 15%-20%, preferably at 20%, of air saturation for 2-3 hours, preferably for 2 hours.

In yet further embodiments, the pulsed hyperoxygenation phase further comprises a step comprising at least four third hyperoxygenation cycles of varying dissolved oxygen levels, wherein each third hyperoxygenation cycle comprises:
(i) maintaining dissolved oxygen at 85%-95%, preferably at 90%, of air saturation for 4-5 hours, preferably for 4 hours, followed by
(ii) maintaining dissolved oxygen at 15%-20%, preferably at 20%, of air saturation for 2-3 hours, preferably for 2 hours.

In some embodiments, when the method comprises culturing conditions that include the pulsed hyperoxygenation phase, the second liquid growth medium is enriched with a source of oleic acid before or during a) the first cycle of the first hyperoxygenation cycles, b) the first cycle of the second hyperoxygenation cycles, and/or c) the first cycle of the third hyperoxygenation cycles.

### DESCRIPTION OF DRAWINGS

Fig. 1 shows the increase in times of cordycepin concentration and *Cordyceps militaris* biomass yields depending on which insect biomass additives were used. The secondary Y axis shows the oleic acid content of total fatty acids of corresponding insects.
Fig. 2 shows the impact of addition of insect biomass material into the growth medium during culturing *Cordyceps militaris* fungi on the concentration of cordycepin.

### DETAILED DESCRIPTION OF THE INVENTION

The present invention is further described in detail. In particular, a method for culturing *Cordyceps* fungi producing cordycepin is provided, wherein the method comprises at least the steps: a) obtaining a fungi inoculum, b) optionally, inoculating the fungi inoculum in a first liquid growth medium, c) inoculating fungi inoculum into a second liquid growth medium, d) culturing under culturing conditions, wherein: (i) before or during step d) the second liquid growth medium is enriched with a source of oleic acid, and/or (ii) culturing conditions include a multiple-stage dissolved oxygen control strategy, and/or (iii) the *Cordyceps* species is a variant of *Cordyceps* species that has reduced capability to synthesize saturated fatty acids. As used herein, terms "culturing", "cultivating", "propagating" are used interchangeably and refer to the process of growing the fungi mycelium, up to the amounts suitable for harvesting the mycelium.

In some embodiments, in the method for culturing *Cordyceps* species, the *Cordyceps* species is selected from *Cordyceps militaris, Cordyceps sinensis* (*Ophiocordyceps sinensis*), *Cordyceps cardinalis, Cordyceps cicadae, Cordyceps kyushuensis* and *Cordyceps bassiana.* In preferred embodiments the *Cordyceps* species is *Cordyceps militaris.* However, other *Cordyceps* fungi species may be as well adapted to cultivation method as provided herein. This can be done, for example, by adapting specific conditions (temperature, medium, etc.) for optimal growth and bioactive compound production required by said species.

The method according to the present invention comprises a step a) of obtaining a fungi inoculum. As such, it will be understood that in order to obtain a fungi inoculum, a skilled person may obtain the *Cordyceps* fungi spores or mycelium and inoculate them on a solid growing medium, such as, e.g. agar, for example, on a potato dextrose agar plate, and incubate at growing conditions that activate and promote growth of inoculum. For example, incubation may take 5 to 10 days (e.g. 7 days) at a temperature of 26-28°C (e.g. 25°C). For faster activation the spores or mycelium may be incubated in the dark. Once the spores or mycelium has been activated, the resulting inoculum may be harvested and inoculated in a first liquid growth medium (step b), for example, in a container such as a flask. The resulting inoculate may be incubated further at growing conditions (at a certain temperature and for a required time period). For example, growing conditions may include incubation at 25°C for 3-5 days. In some embodiments, the first liquid growth medium is the same as the second growth medium; for example the first liquid growing medium may have the same composition as the second liquid growth medium that is added before the step d).

The method of the present invention further comprises the step c) inoculating fungi inoculum into the second liquid growth medium. In this step the fungi inoculum from step a) or step b) added/inoculated into a growth medium where the mycelium will be grown up until its harvesting. The second liquid growth medium may comprise the components that are needed from *Cordyceps* fungi growing. A skilled person working in the field of fungi cultivation and in the field of bioreactors in general, would be aware or would have appropriate manuals that provide growing medium compositions for growing/culturing *Cordyceps* fungi.

The method of the present invention further provides a step d) culturing under culturing conditions. In general, the culturing conditions include temperature, time and medium that are known to the person skilled in art as needed for cultivating *Cordyceps* fungi. For example, the culturing under culturing conditions may include growing of *Cordyceps* fungi in a bioreactor. In some embodiments, the culturing conditions include growing fungi for about 10 to 15 days. The present invention disclosure further provides advantageous improvements of the method for culturing *Cordyceps* fungi producing cordycepin. When any of the below described improvements of the method are used, alone, or in combination, the *Cordyceps* fungi that is cultured produces high amounts and/or high concentrations cordycepin and the yield of *Cordyceps* biomass is increased.

One of the advantageous improvements provided herein is enriching the second growth medium with a source of oleic acid. Oleic acid may function as an enhancer or regulator of the genes involved in cordycepin production. The source of oleic acid can be added to the second growth medium before or during the step d) culturing under culturing conditions. In some embodiments, the enrichment with a source of oleic acid is added after an exponential growth phase. Preferably, the enrichment with a source of oleic acid is added more than one time, e.g. 2, 3, 4, 5, 6, 7, 8, 9, 10 times. For example, the enrichment with a source of oleic acid may take place every day after the exponential growth phase. In some embodiments, an amount of a source of oleic acid is added that results in concentration of oleic acid in the liquid growth medium from about 110 mg/L to about 155 mg/L. In preferred embodiments, the source of oleic acid is insect-derived material. The insect material may be selected from the group consisting of insect biomass, insect biomass powder, insect extracts and insect homogenates. Insect biomass generally refers to a biomass of a dried insect. In preferred embodiments, the insect material is selected from insects comprising from 5% (w/w) to 65% (w/w) oleic acid content of total fatty acids. Preferably, insect material comprising from 20% to 45% (w/w) oleic acid content of total fatty acids may be used. In some embodiments, insect material comprising 20%, 25%, 30%, 35%, 40%, 45% or more % (w/w) oleic acid content of total fatty acids is used.

In some embodiments, the insect material is from insect family selected from the group consisting of *Tenebrionidae, Gryllidae, Acrididae, Acrididae, Bombycidae, Saturniidae, Formicidae, Scarabaeidae, Pyrrhocoridae, Carabidae, Lucanidae* insect families or combination thereof. In some embodiments, the insect material is from insect genus selected from *Tenebrio, Acheta, Schistocerca, Chorthippus, Bombyx, Cricula, Formica, Protaetia, Dysdercus, Antheraea, Calosoma, Dorcus,* or combination thereof. In further embodiments, the insect material is from insect selected from *Tenebrio molitor, Acheta domesticus, Schistocerca gregaria, Chorthippus brunneus, Bombyx mori, Cricula trifenestrata, Formica rufa, Protaetia brevitarsis, Dysdercus koenigii, Antheraea pernyi, Calosoma scrutator, Dorcus hopei,* or combination thereof. For example, the insect material is from insects selected from *Tenebrionidae, Gryllidae, Acrididae, Acrididae, Bombycidae, Saturniidae, Formicidae* or combination thereof. In other examples, the insect material is from insects selected from *Scarabaeidae, Pyrrhocoridae, Carabidae, Lucanidae* insect families or combination thereof. The insect material from these insect sources can be additionally advantageous, as they are known to have oleic acid content of total fatty acids as high as, e.g. 50% or 60% (Vesović et al., 2023; Yeo et al., 2013). As such, *Scarabaeidae* and *Pyrrhocoridae* could be used.

In some embodiments, an insect material comprising a combination of insect materials is used. For example, two or more insect species can be combined. In one preferred embodiment, the insect material comprises insects from *Tenebrio molitor* and *Acheta domesticus.* When a combination of insects is used in the insect material, various ratios can be used. In some examples, in case of 2 insect combination, a 1:1 weight ratio is used, in other example different weight ratios can be used, for example 1:2, 1:3, 1:5.

Another advantageous improvement in the method of the present invention is achieved when the method comprises culturing conditions that include the pulsed hyperoxygenation phase. In some embodiments, the pulsed hyperoxygenation phase comprises a step comprising at least four first hyperoxygenation cycles of varying dissolved oxygen levels, wherein each first hyperoxygenation cycle comprises:
(i) maintaining dissolved oxygen at 45%-55%, preferably at 50%, of air saturation for 4-5 hours, preferably for 4 hours, followed by
(ii) maintaining dissolved oxygen at 15%-20%, preferably at 20%, of air saturation for 2-3 hours, preferably for 2 hours.

In further embodiments, the step comprising at least four first hyperoxygenation cycles of varying dissolved oxygen levels is followed by a step comprising at least four second hyperoxygenation cycles of varying dissolved oxygen levels, wherein each second hyperoxygenation cycle comprises:
(i) maintaining dissolved oxygen at 65%-75%, preferably at 70%, of air saturation for 4-5 hours, preferably for 4 hours, followed by
(ii) maintaining dissolved oxygen at 15%-20%, preferably at 20%, of air saturation for 2-3 hours, preferably for 2 hours.

In yet further embodiments, the step comprising at least four first hyperoxygenation cycles of varying dissolved oxygen levels is followed by a step comprising at least four third hyperoxygenation cycles of varying dissolved oxygen levels, wherein each third hyperoxygenation cycle comprises:
(i) maintaining dissolved oxygen at 85%-95%, preferably at 90%, of air saturation for 4-5 hours, preferably for 4 hours, followed by
(ii) maintaining dissolved oxygen at 15%-20%, preferably at 20%, of air saturation for 2-3 hours, preferably for 2 hours.

In some embodiments, when the method comprises culturing conditions that include the pulsed hyperoxygenation phase, the second liquid growth medium is enriched with a source of oleic acid before or during a) the first cycle of the first hyperoxygenation cycles, b) the first cycle of the second hyperoxygenation cycles, and/or c) the first cycle of the third hyperoxygenation cycles.

In some embodiments, the culturing conditions comprise the following growing phases: a hyphal initiation phase, an exponential growth phase, a pulsed hyperoxygenation phase, a synthesis stabilization phase. In some embodiments, the culturing conditions comprise a hyphal initiation phase, an exponential growth phase and an extended exponential growth phase.

In some embodiments, during the hyphal initiation phase, the level of dissolved oxygen is maintained at 5-8% of air saturation. For example, the hyphal initiation phase may take 1-2 days.

In some embodiments, during the exponential growth phase, where the level of dissolved oxygen is maintained at 25-30 % of air saturation. For example, the exponential growth phase may take 7-9 days.

In some embodiments, the pulsed hyperoxygenation phase comprises the steps comprising the first, second and third hyperoxygenation cycles as described above. For example, the pulsed hyperoxygenation phase may take 1-3 days.

In some embodiments, during the synthesis stabilization phase, the level of dissolved oxygen is maintained at 15-20 % of air saturation. For example, the synthesis stabilization phase may take 3-5 days.

Yet another advantageous improvement as provided in the present invention disclosure, is a method for culturing *Cordyceps* fungi producing cordycepin, wherein the *Cordyceps* species is a variant of *Cordyceps* species that has reduced capability to synthesize saturated fatty acids. As a result such a variant of *Cordyceps* species produces higher cordycepin amounts during culturing. In some embodiments, a mutant *Cordyceps* species can be used, for example a mutated *Cordyceps* militaris strain. For example, a Δfas1 mutant strain of *Cordyceps* species can be used. As used herein, Δfas1 mutant refers to a mutant strain in which at least a part or a whole gene fas1 has been deleted or inactivated by a mutation. A Δfas1 mutant is unable to synthesize saturated fatty acids, such as palmitic acid (C16:0) or stearic acid (C18:0), which allows redirection of acetyl-CoA metabolism toward cordycepin biosynthesis (Li et al., 2024; Wang et al., 2022).

As further provided herein, the method of the present invention may further comprise a step of harvesting the cultured *Cordyceps* fungi mycelium. This may be done, for example, after the step d), once the *Cordyceps* species has been cultured to produce sufficient or desirable amounts of cordycepin (for example, when the total amount of cordycepin does not accumulate over the time anymore) or *Cordyceps* fungi biomass. The harvested product may be dried or freeze-dried. In preferred embodiments, after harvesting, cordycepin is extracted. Various options and protocols for extraction and/or purification of cordycepin are available in the art.

### EXAMPLES

### Methods

### Protocol for Fatty Acid Profiling

The following materials were utilized for fluorescence resonance energy transfer (FRET) analysis:
(i) Insect: *Tenebrio molitor* (mealworm), *Schistocerca gregaria* (locust), *Bombyx mori* (silkworm), and other tested species.
(ii) Fluorescent probes: NBD-X-labeled oleic acid (*Molecular Depot (Catalog Number: B2011458),* NBD-OA, excitation/emission = 467/535 nm).
(iii)Binding protein: Fatty acid-free bovine serum albumin (BSA, Sigma-Aldrich A7030).
(iv) Buffers: 10 mM phosphate-buffered saline (PBS, pH 7.4) with 0.01% sodium azide.
   1. Sample Preparation
      1.1 Lipid Extraction. Insect biomass (100 mg DW) was homogenized in liquid nitrogen and subjected to Folch extraction (CHCl₃:MeOH, 2:1 v/v). Total lipids were saponified with 0.5M KOH-MeOH (70°C, 1 hr) and acid-liberated (pH 2.0 HCl).
      1.2 Fluorescent Labeling. Free fatty acids (FFAs) were reacted with NBD-X succinimide ester (Biomol GmbH (catalog number: ABD-829), 1:5 molar ratio) in anhydrous DMSO (37°C, 2 hr, dark). Labeled FFAs (NBD-FA) were purified via C18 SPE (Waters Sep-Pak^{®}).
   2. FRET Assay Configuration. A competitive binding assay was implemented using BSA's intrinsic tryptophan (donor) and NBD (acceptor). Stepwise Procedure contained Pre-complex Formation (10 µM BSA + 50 µM NBD-OA were incubated (15 min, 25°C) in PBS), Competitive Displacement (Insect FFAs (0-100 µg/mL) were introduced to displace NBD-OA from BSA), and Detection. For detection fluorescence was measured at excitation = 280 nm (tryptophan), emission = 535 nm (NBD), and FRET efficiency (E) was calculated as: $E = 1 - \frac{{I}_{DA}}{{I}_{D}}$ Where I_{DA} is donor intensity with acceptor, I_{D} is donor alone.
   3. Quantification and Validation. Calibration Curve: Oleic acid standards (0-100 µg/mL) were analyzed to establish linear regression. Cross-Validation: results were compared to GC-FID (AOAC 991.39) for 15 insect samples, showing 92.4 ± 3.7% agreement. Species-Specific Adjustments: in case of Coleoptera 0.1% Triton X-100 was added to disrupt chitin-bound lipids; in case of Lepidoptera saponification extended to 2 h for wax ester hydrolysis.

### Cordyceps militaris cultivation conditions

Primary Inoculum Preparation. Selected *Cordyceps militaris* strain (DSM23612 (Leibniz Institute, DSMZ-German Collection of Microorganisms and Cell Cultures GmbH) was activated by transferring from storage to fresh potato dextrose agar (PDA) plates and incubating at 25 °C. After 7 days of growth, the mycelium was aseptically harvested and fragmented in sterile water containing 0.1% (v/v) Tween 80. The mycelial suspension was then transferred to seed medium (same composition as initial growth phase medium: (Sucrose (Merck Millipore, Catalog Number: 1037891000) at 27 g/L; Casein hydrolysate (Merck Millipore, Catalog Number: 1022450500) at 10 g/L; KH₂PO₄ (Merck Millipore, Catalog Number: 1048711000) at 1 g/L; MgSO₄·7H₂O (Merck Millipore, Catalog Number: 1058860500) at 0.5 g/L ) in 1L Erlenmeyer flasks with a working volume of 300 mL. The seed flasks were incubated on an orbital shaker at 160 rpm and 25°C for 5 days.

Bioreactor Cultivation Process. The bioreactor cultivation followed a multi-phase process with precisely controlled environmental parameters:
Phase 1: Hyphal Initiation Phase (Days 0-2). The sterilized bioreactor containing initial growth phase medium was inoculated with 5% (v/v) of the primary inoculum. Cultivation conditions were set as follows: 25°C, pH 6.0 (controlled automatically), Dissolved oxygen (DO) was maintained at 5-8% of air saturation to mimic natural soil conditions, agitation was initially 200 rpm, and was adjusted as needed to maintain DO levels. During this phase, the focus is on initial hyphal development and adaptation.
Phase 2: Exponential Growth Phase (Days 2-9). Cultivation conditions were adjusted as follows: temperature was maintained at 23°C, pH was adjusted to 5.8 and controlled at this level, DO was increased and maintained at 25-30% of air saturation to induce oxidative stress for metabolite production, agitation was adjusted based on DO demand. This phase focuses on maximizing biomass production while establishing a robust mycelial network. Cordycepin level is measured on day 5 and daily thereafter.
Phase 3: Pulsed Hyperoxygenation Phase (Days 9-11). The DO control strategy implemented a gradual ramping pattern:
   Day 9: DO increased from 30% to 50% O₂ over 6h, thus a regime comprises: DO maintained at 50% for 4h, then reduced to 20% for 2h; such a regime/cycle is repeated 4 times;
   Day 10: DO increased from 50% to 70% O₂ over 6h, thus a regime comprises: DO maintained at 70% for 4h, then reduced to 20% for 2h; such a regime/cycle is repeated 4 times;
   Day 11: DO increased from 70% to 90% O₂ over 6h, thus a regime comprises: DO maintained at 90% for 2h, then reduced to 20% for 4h; such a regime/cycle is repeated 4 times.

This pulsed hyperoxygenation enhances NADPH oxidase activity. Cultivation temperature was maintained at 23°C, pH was controlled at 5.8, and agitation was adjusted automatically to maintain the DO setpoints.

On day 9, supplementary feeding was initiated through pulse addition of concentrated nutrients:
Glucose (Sigma-Aldrich, Catalog Number G7021-1KG) (500 g/L): added once daily (10 mL per liter of working volume);
Adenosine (Sigma-Aldrich, Catalog Number A9251) (50 g/L): added once daily (10 mL per liter of working volume);
Hydrolyzed insect powder (Thailand Unique JR Unique Foods Ltd.), aqueous solution (300 g/L): added once daily (5 mL per liter of working volume).

Phase 4: Synthesis Stabilization Phase (Days 11-15). DO was reduced and maintained at 15% of air saturation to protect ROS-sensitive enzymes. On day 13, metabolic elicitors were introduced to maximize cordycepin production: Ammonium sulfate (Sigma-Aldrich, Catalog Number 1012165000) (added to a final concentration of 2 g/L), Manganese sulfate (Merck Millipore, Catalog Number 105941) (supplemented to increase concentration by 0.02 g/L).

Cultivation conditions were: temperature was reduced to 20°C, pH was maintained at 5.8. This phase focuses on channelling cellular metabolism toward cordycepin production and maintaining enzyme stability for optimal yield

The specific cordycepin formation (production) rate (qₚ), expressed in mg/L/day, was calculated as qₚ = ΔC_{cordycepin} / Δt; where ΔC_{cordycepin} is change in cordycepin concentration (mg per Litre (mg/L) of liquid growth medium), and Δt is time interval (days).

In cases where insect biomass additives were not used, addition of insect powder solution step was omitted. In cases where hyperoxygenation steps were not used, Phase 3 (Production Phase), Phase 4 (Enhancement Phase), Pulsed Oxygenation Cycle steps were omitted.

### Quantitative Analysis of Cordycepin in Cordyceps militaris Powder via High-Performance Liquid Chromatography (HPLC)

Cordycepin concentration milligrams per gram of dry weight (mg/g DW) of *Cordyceps* militaris is determined as follows.

Sample preparation. *Cordyceps* militaris biomass is dried at 60°C in a convection oven until constant mass is achieved. The dried material is ground to a fine powder using a mechanical mill (particle size < 250 µm) and stored in airtight containers at -20°C until analysis. Solvent Extraction 1. Extraction Process: 1.00 g of powdered sample is combined with 10 mL of 80% methanol (v/v) in a 50 mL polypropylene centrifuge tube. 2. Maceration: The mixture is agitated at 150 rpm for 24 hours at 25°C using an orbital shaker. 3. Phase Separation: The suspension is centrifuged at 4,500 rpm (2,500 × g) for 5 minutes in a refrigerated centrifuge maintained at 4 °C. 4. Filtrate Preparation: The supernatant is decanted and filtered through a 0.45 µm PTFE membrane filter under vacuum. 5. Concentration: The filtrate is evaporated to dryness and reconstituted in 1 mL of HPLC-grade methanol to yield a final concentration of 10 mg/mL.

Chromatographic Analysis. Extracts were analyzed by HPLC (RP-C18 column, 260 nm detection, 0.1% H₃PO₄:MeOH = 85:15 mobile phase). Agilent 1260 Infinity HPLC System was used.

### Protocol for Developing UV-Mutagenized Cordyceps Strains with Enhanced Oleic Acid Metabolism and Cordycepin Production

### 1. Strain Preparation and Spore Collection

1.1 Cultivation of Parental *Cordyceps* militaris Strains. Wild-type *Cordyceps* militaris strains were inoculated on Potato Dextrose Agar (PDA) supplemented with 2% peptone and 0.5% yeast extract. Cultures were incubated at 22°C under 16:8 hr light:dark cycles for 14 days to induce sporulation. Spores were harvested using a sterile scraper and suspended in 0.85% NaCl solution containing 0.1% Tween 80 to achieve a concentration of 10⁶-10⁷ spores/mL.

1.2 Protoplast Isolation (Optional for Hybridization). For strains requiring somatic hybridization prior to mutagenesis: Mycelia were digested in protoplasting buffer (0.7 M NaCl, 0.2 M CaCl₂, 20 mg/mL Novozyme 234, pH 5.8-6.0) at 20°C for 6 hr. The mixture was centrifuged at 3,000 × g for 10 min, and protoplasts were resuspended in fusion buffer (0.05 M phosphate buffer, 30% PEG 6000, 50 mM CaCl₂). The suspension was incubated for 20 min to facilitate fusion. Fused protoplasts were plated on Complete Medium Agar (CMA), and hybrids were selected based on colony morphology.

### 2. UV Mutagenesis Protocol

2.1 UV Exposure Parameters. A 10 mL spore suspension was transferred to a sterile Petri dish (depth ≤3 mm). The suspension was irradiated under UV-C (254 nm) at 50 cm distance for 60-80 min with constant agitation. Dosage calibration: 0.5-1.0 J/m²/sec was applied to achieve 70-90% spore lethality. To prevent photoreactivation, the suspension was wrapped in foil and incubated at 20°C for 60 min in darkness.

2.2 Post-Mutagenesis Recovery. The irradiated suspension was diluted 10-fold in sterile saline. Aliquots (100 µL) were plated on oleic acid-supplemented agar (OAA): Base medium: 20 g/L glucose, 5 g/L NH₄NO₃, 1 g/L KH₂PO₄, 0.5 g/L MgSO₄·7H₂O; Supplement: 1.5% (v/v) oleic acid emulsified with 0.1% Tween 80; Agar: 1.8% (w/v), pH 6.0. Plates were incubated at 22°C for 7 days.

### 3. Mutant Selection and Screening

3.1 Primary Screening on Oleic Acid Agar. Fast-growing colonies (≥2× radial growth vs. wild-type) on OAA were identified. Promising mutants were subcultured on OAA for three generations to ensure phenotypic stability.

3.2 Secondary Screening in Liquid Fermentation. Selected mutants were inoculated in oleic acid broth (OAB): 30 g/L glucose, 10 g/L peptone, 5 g/L yeast extract, 2% (v/v) oleic acid. 150 mL medium was incubated in 500 mL baffled flasks, at 22°C, 150 rpm, for 12-day fermentation. Mycelia were harvested at 48-hr intervals for biomass (dry weight) and cordycepin analysis.

3.3 Cordycepin Quantification. Cordycepin was extracted via ultrasonication in 70% ethanol (1:20 w/v, 40 kHz, 30 min). Extracts were analyzed by HPLC (C18 column, 260 nm detection, 0.1% H₃PO₄:MeOH = 85:15 mobile phase). Mutants showing ≥1.5× cordycepin yield vs. parental strains were selected.

### 4. Genetic and Metabolic Characterization

4.1 Molecular Validation of Mutants. RAPD-PCR was performed using primers OPA-02 (5'-TGCCGAGCTG-3', SEQ ID NO: 1) and OPB-07 (5'-GGTGACGCAG-3', SEQ ID NO: 2) to confirm genomic alterations. rDNA-ITS regions were sequenced using Applied Biosystems 3500xl Genetic Analyzer (Thermo Fisher Scientific) to verify species integrity and exclude contamination.

4.2 fas1 Gene Deletion Screening. Genomic DNA was extracted using the CTAB method (Schenk et al., 2023). The fas1 locus was amplified with primers Fas1-F (5'-ATGGCGATCCTCAACGAC-3', SEQ ID NO: 3) and Fas1-R (5'-TCAGGCCTTGTAGTCCAGG-3', SEQ ID NO: 4). Δfas1 mutants were identified via: PCR product size shift: Wild-type = 2.1 kb, Δfas1 = 1.3 kb (if replaced by selection marker). Fatty acid profiling: Δfas1 mutants exhibited ≥60% reduction in C16:0 and C18:0 fatty acids.

### Results

### Example 1. The oleic acid contents of various insect biomass

Table 1 shows the oleic acid content in the insect biomass used in the present examples.

**Table 1**

| Sample No. | **Common Name** | **Latin Name** | **Oleic Acid Content (% (w/w) of total fatty acids)** |
|---|---|---|---|
| **A2** | Mealworms | *Tenebrio molitor* | ~40.83% |
| **A3** | House crickets | *Acheta domesticus* | 23-27% |
| **A4** | Mixed mealworm-cricket | *T. molitor+A. domesticus* | 30-35% (estimated blend) |
| **A5** | Locust | *Schistocerca gregaria* | ~40.7% |
| **A6** | Grasshopper | *Chorthippus brunneus* | ~25.1% |
| **A7** | Silkworm | *Bombyx mori* | ~34.97% |
| **A9** | Green silkworm variant | *Cricula trifenestrata* | ~34-36% (estimated) |
| **A10** | Red wood ant | *Formica rufa* | No data available |

Species with high in oleic acid content (40% and more):
Mealworms (*Tenebrio molitor*): At approximately 40.83% oleic acid, mealworms show remarkably high content. Research indicates that "oleic acid (C18:1) formed the major lipid component in 40.83%, which was followed by linoleic acid (C18:2, omega-6 fatty acid) with 29.80%". This makes mealworms an excellent candidate for oleic acid-rich media formulations.

Locust (*Schistocerca gregaria*): With approximately 40.7% oleic acid content, locusts nearly match mealworms.

Species with medium oleic acid content (30-40%):
Silkworm (*Bombyx mori*) contain approximately 34.97% oleic acid.

Mixed mealworm-cricket: This blend (50/50 by mass) would theoretically contain an intermediate oleic acid content between its component species (estimated 30-35%), potentially offering balanced fatty acid profiles.

Species with lower oleic acid content (< 30%):
House crickets (*Acheta domesticus*) - with 23-27% oleic acid, crickets contain less oleic acid than mealworms but still offer a significant amount.

Grasshopper (*Chorthippus brunneus*) contain approximately 25.1% oleic acid, this is considered modest amount.

### Example 2. Impact of various insect biomass additives on Cordyceps mycelium biomass and cordycepin yields

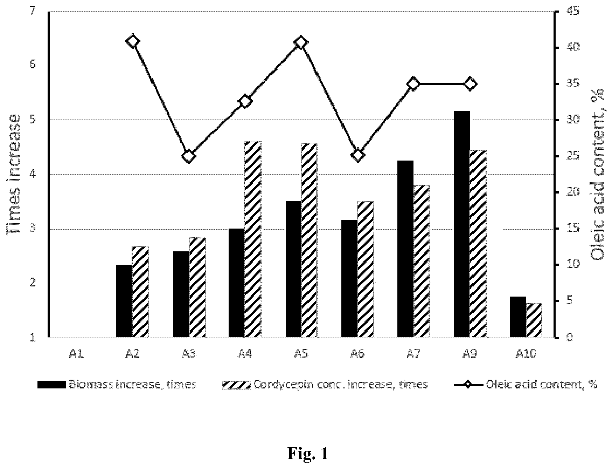

As can be seen from the Table 2 and Fig. 1, when varying insect biomass was added into the growing medium, cordycepin concentration was increased from 1.6 to 4.6 times, compared to the control sample (A1). The most effective insect biomass additives for cordycepin concentration increase were mixed mealworm-cricket (A4; 4.6× increase), locust (A5; 4.5× increase), green silkworm variant (A9; 4.4× increase), silkworm (A7; 3.8× increase). Additionally, the results where the grasshopper biomass was used stand out, as grasshopper is considered to have lower oleic acid content, but the cordycepin concentration increase (A6; 3.5× increase) was comparable to that achieved with insect biomasses with higher oleic acid content.

**Table 2.**

| Sample No. | Common Name | Latin name | Biomass yield (g) | Cordycepin conc. (mg/g DW) |
|---|---|---|---|---|
| A1 | No additives / Control | | 1.2 ± 0.3 | 13.04 |
| A2 | Mealworms | *Tenebrio molitor* | 2.8 ± 0.4 | 34.86 |
| A3 | House crickets | *Acheta domesticus* | 3.1 ± 0.5 | 36.95 |
| A4 | Mixed mealworm-cricket | *Tenebrio molitor + Acheta domesticus* | 3.6 ± 0.6 | **60.1** |
| A5 | locust | *Schistocerca gregaria* | 4.2 ± 0.7 | 59.64 |
| A6 | grasshopper | *Chorthippus brunneus* | 3.8 ± 0.5 | 45.71 |
| A7 | Silkworm | *Bombyx mori* | 5.1 ± 0.9 | **49.61** |
| A9 | Green silkworm variant | *Cricula trifenestrata* | 6.2 ± 1.1 | **58.0** |
| A10 | Red wood ant | *Formica rufa* | 2.1 ± 0.4 | 21.32 |

Effect on *Cordyceps* mycelium biomass. All insect additives increased biomass yield compared to the control (Table 2). The best yields of *Cordyceps* militaris mycelium were Green silkworm variant (A9; 5.1× increase), silkworm (A7; 4.2x increase), locust (A5; 3.5× increase), grasshopper (A6; 3.2× increase).

High *Cordyceps* mycelium biomass yield is relevant for several important reasons:
1) Total cordycepin output - even with identical cordycepin concentration, higher biomass yield directly increases the total cordycepin produced per batch.
2) Process economics - higher yields from the same inputs improve the economic efficiency of the production process.
3) Bioactive compound diversity - *Cordyceps* contains numerous bioactive compounds beyond cordycepin that may have commercial value. The experimental data shows significant biomass increases with certain insect additives - the green silkworm variant produced 5.1× more biomass than the control.
4) Scale-up considerations - for industrial scaling, consistent and high biomass yields are essential for reliable manufacturing processes.
5) Substrate utilization efficiency - higher biomass yield indicates more efficient conversion of nutritional inputs, reducing waste and improving sustainability.

The increase of both, Cordycepin concentration and *Cordyceps* mycelium biomass is important in several aspects of the cultivation process:
1) Economic efficiency: Higher cordycepin concentration per gram of biomass translates to more efficient extraction processes and potentially lower production costs. The data shows significant variations in this ratio depending on the insect additive used.
2) Comparative evaluation: This ratio provides the clearest measure for comparing the effectiveness of different insect additives in enhancing cordycepin production
3) Commercial viability: For industrial applications, maximizing the amount of cordycepin per *Cordyceps* mycelium biomass is essential to achieve cost-effective production of pharmaceutical-grade cordycepin.

The data reveals some surprising relationships between *Cordyceps* fungi and various insect species:
1) In case of the mixed mealworm-cricket combination (A4), while the *Cordyceps* mycelium biomass yield remained similar to the yield when each of the insect biomasses were used separately, the combination (A4) produced higher cordycepin concentration better results than when either of the insect biomasses were used alone. As a result, the total amount of cordycepin produced using the combination (A4) was higher than the sum amount of cordycepin produced using mealworm biomass (A2) and cricket biomass (A3), thus suggesting a synergistic effect.
2) Surprising compatibility - the results showed that insects like locusts and grasshoppers, which may not be natural hosts for *Cordyceps* fungi, significantly enhanced both cordycepin production and *Cordyceps* mycelium biomass yield.
3) Silkworm efficiency: Both silkworm variants (A7, A9) demonstrated excellent performance, indicating strong compatibility with *Cordyceps* fungi.

### Example 3. Use of hyperoxygenation step during Cordyceps mycelium growth

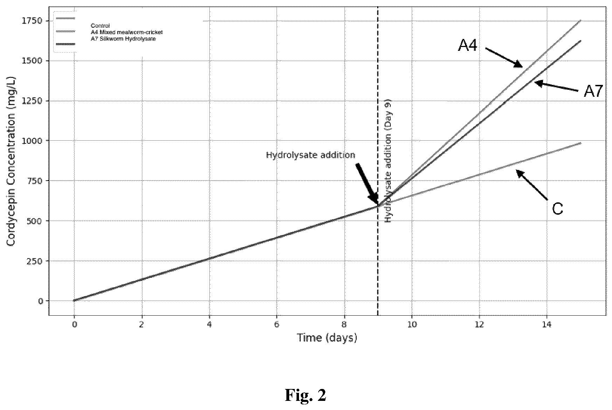

Table 3 and Fig. 2 reflects the time course of cordycepin production, where the cordycepin concentration (mg/L of liquid medium) is shown in cases where insect biomass additives were used and in cases where the insect biomass additives were not used.

**Table 3**

| **Time Period** | **Control (mg/L)** | **A4 Mixed mealworm-cricket (mg/L)** | **A7: Silkworm larvae (mg/L)** | **Percentage Increase vs. Control (A4/A7)** |
|---|---|---|---|---|
| **Day 0-9 (Baseline)** | Linear increase | Linear increase | Linear increase | - |
| **Day 10 (24h post-supplementation)** | 589.4 ± 25.1 | 642.8 ± 28.3 | 623.6 ± 26.7 | +9.1%/+5.8% |
| **Days 13-15 (Enhancement phase)** | 982.4 ± 42.6 | 1748.7 ± 68.3 | 1621.0 ± 54.1 | +78.0%/+65.0% |
| **Production Rate (Days 9-15)** | 65.5 mg/L/da y | 184.2 mg/L/day | 166.3 mg/L/day | +181%/+154% |
| **Final Concentration (Day 15)** | 982.4 ± 42.6 | 1748.7 ± 68.3 | 1621.0 ± 54.1 | - |

The cordycepin production in mg/L of the liquid growth medium as shown in Table 1 was determined by HPLC analysis as described above.

The cordycepin production profiles show that significant differences between treatments began to appear approximately 24 hours after supplementation with insect hydrolysates (day 10). Prior to supplementation, all cultures showed similar cordycepin production rates. This observation confirms that the enhanced production is directly related to the insect hydrolysate addition rather than random variation.

Table 4 shows results across different experimental conditions. As can be seen, use of hyperoxygenation alone significantly increased both, cordycepin concentration as well as *Cordyceps* mycelium biomass yield. When combined with insect biomass additive (in case of Table 3 it was the mealworm-cricket combination A4), cordycepin concentration was increased almost 8 times, compared to control.

**Table 4 Biomass Yield and Cordycepin Concentration Across Experimental Conditions**

| Experimental Condition | Biomass Yield (g DW) | Cordycepin Conc. (mg/g DW) | Cordycepin Productivity (mg/L/day*) |
|---|---|---|---|
| Control | 1.2±0.31 | 13.04±1.2 | 65.56 |
| *Cordyceps* + A4 Supplement | 3.6±0.63 | 60.1±2.8 | 184.21 |
| *Cordyceps +* Hyperoxygenation | 4.8±0.94 | 41.3±1.9 | 142.61 |
| *Cordyceps* + A4 + Hyperoxygenation | 5.7±1.15 | 102.4±4.1 | 265.82 |

| | | | |
|---|---|---|---|
| * - Cordycepin productivity (mg/L/day) is calculated through daily sampling. 10 mL liquid mycelium was collected every 24 hours during fermentation. | | | |

The presently provided hyperoxygenation method introduces three new components that have not been employed in previous research.

### 1) Phase-Specific Oxygen Profiling

The four-phase modulation strategy represents a significant advancement over conventional two-stage approaches (as, e.g. the one used by (Mao & Zhong, 2004)):
(i) Hyphal initiation at 5-8% O₂ mimics natural soil conditions;
(ii) Exponential growth at 25-30% O₂ induces oxidative stress for metabolite production;
(iii) Pulsed hyperoxygenation (50-90% O₂ cycles) during Days 9-11 enhances NADPH oxidase activity;
(iv) Synthesis stabilization at 15% O₂ protects ROS-sensitive enzymes.

No prior studies have implemented this multi-phase oxygen regimen with biological rationale for each transition point.

2) Pulsed Oxygenation Technology provides a different approach compared to gradual DO reductions in prior art.

As presently provided, pulsed oxygenation technology implements a three-day cyclic ramping protocol (Days 9-11) that diverges fundamentally from prior gradual DO reduction strategies. This approach combines controlled oxidative stress induction with NADPH oxidase optimization through daily oxygen modulation cycles:
Day 9: Dissolved oxygen (DO) undergoes a cyclic ramp from 30% → 50% O₂ over 6 hours, maintains 50% for 4 hours, then reduces to 20% for 2 hours.

Day 10: A repeated cycle ramps DO from 50% → 70% O₂ over 6 hours, holds at 70% for 4 hours, then lowers to 20% for 2 hours.

Day 11: The final cycle elevates DO from 70% → 90% O₂ over 6 hours, sustains 90% for 2 hours, then decreases to 20% for 4 hours.
during Days 9-11 demonstrate unique physiological manipulation:
(i) Increase cordycepin production rates by 181% vs control;
(ii) Generate 45.3% higher yields than constant DO strategies;
(iii) Work in synergy with insect-enriched media to achieve as high as 1748.7 mg/L final cordycepin concentration.

This pulsed approach differs fundamentally from the gradual DO reductions in literature24.

### 3) Integration with oleic acid metabolism

The oxygen protocol explicitly interacts with insect-supplemented media through:
(i) Enhanced fatty acid β-oxidation under high O₂ conditions;
(ii) ROS-mediated upregulation of cordycepin biosynthetic genes;
(iii) Stabilization of oxygen-sensitive desaturases during late phases;

This biochemical integration represents an unexpected synergy between oxygenation and lipid metabolism.

**Table 5. Comparative Performance Analysis**

| Parameter | Literature methods * | The method of the present invention | Improvement level |
|---|---|---|---|
| Max Cordycepin (mg/L) | 2214.5 | 1748.7 | -21 % |
| Productivity (mg/L/day) | 15.5 | 184.2 | 1088 % |
| Process Duration (days) | 24 | 15** | -37.5% |
| Oxygen Complexity | 2-stage | 4-phase + pulses | 100 % |

| | | | |
|---|---|---|---|
| *(Mao & Zhong, 2004; Zhang & Liu, 2016) **Productivity was calculated starting from Day 9. | | | |

During the enhancement phase (days 13-15), the rate of cordycepin production increased dramatically in all treatments, but the effect was most pronounced in the Mixed mealworm-cricket (A4) and silkworm (A7) supplemented cultures. The final cordycepin concentration after 15 days reached 1748.7 mg/L with silkworm hydrolysate supplementation, which is 78.0% higher than the control and represents one of the highest reported cordycepin titres from C. militaris cultivation.

The described hyperoxygenation method introduces novel operational parameters (phase-specific levels, pulsed cycles) and biological rationales (ROS enzyme stabilization, NADPH optimization) not found in prior cordycepin research. While building on established principles of oxygen influence on fungal metabolism, its specific implementation constitutes an improvement step beyond existing two-stage DO control strategies. The integration with insect-derived oleic acid substrates further distinguishes this approach from conventional dissolved oxygen optimization studies. The combination of A4 Mixed mealworm-cricket supplementation with the multiple phase DO control strategy achieved a remarkable 78.0% increase in cordycepin production compared to the conventional cultivation method. This synergistic effect represents a significant advancement in biotechnological production of cordycepin.

### Example 4. Characterization of UV-Mutagenized Cordyceps Strains

A total of 12 stable mutant strains were isolated and validated through the UV mutagenesis protocol. These included: 3 primary candidates with ≥2× cordycepin yield (selected from 50 screened colonies), and 9 secondary mutants showing 1.5-1.8× increased productivity.

The developed mutant strains exhibited the following defining characteristics:
(i) Genetic Profile:
   Deletion of the fas1 gene (Δfas1), confirmed via PCR amplification showing a 1.3 kb fragment instead of the wild-type 2.1 kb fragment.
   Reduced fatty acid synthase activity, redirecting metabolic flux toward cordycepin biosynthesis.
(ii) Metabolic Performance:
   Cordycepin Yield: ≥1.5× higher than parental strains (e.g., 1,748.7 mg/L vs. 982.4 mg/L in controls).
   Fatty Acid Profile: 60% reduction in saturated fatty acids (C16:0, C18:0) compared to wild-type strains.
   Oleic Acid Utilization: Enhanced growth rates (2× radial expansion) on oleic acid-supplemented agar.
(iii) Phenotypic Stability: maintained enhanced traits over three generations of subculturing.

The Δfas1 mutant (the highest-performing strain) was prioritized for scaled cultivation due to its synergistic compatibility with insect-based media and hyperoxygenation protocols.

**Table 6. Performance of Δfas1 Mutants vs. Wild-Type Cordyceps militaris**

| Parameter | Wild-Type Strain | Δfas1 Mutant | Improvement |
|---|---|---|---|
| Cordycepin Yield (mg/L) | 982.4 ± 42.6 | 1,748.7 ± 68.3 | 78 % |
| Biomass Yield (g DW) | 1.2 ± 0.3 | 2.1 ± 0.4 | 75 % |
| Production Rate (mg/L/day) | 65.5 | 184.2 | 181 % |
| C16:0 Fatty Acid (%) | 18.7 ± 1.2 | 7.5 ± 0.8 | -60 % |
| C18:0 Fatty Acid (%) | 12.4 ± 0.9 | 4.9 ± 0.5 | -60 % |
| Oleic Acid Utilization | Baseline | 2× enhanced | 100 % |

### References

1. Thepmalee, C., Jenkham, P., Ramwarungkura, B., Suwannasom, N., Khoothiam, K., Thephinlap, C., Sawasdee, N., Panya, A., & Yenchitsomanus, P. (2024). Enhancing cancer immunotherapy using cordycepin and Cordyceps militaris extract to sensitize cancer cells and modulate immune responses. Scientific Reports, 14(1), 21907. https://doi.org/10.1038/s41598-024-72833-x
2. Chang, Y., Liu, X., Jiao, Y., & Zheng, X. (2024). Improved Cordycepin Production by Cordyceps Militaris Using Corn Steep Liquor Hydrolysate as an Alternative Protein Nitrogen Source. Foods, 13(5), 813. https://doi.org/10.3390/foods13050813
3. Amna, T., Amina, M., Sharma, P. R., Puri, S. C., Al-Youssef, H. M., Al-Taweel, A. M., & Qazi, G. N. (2012). Effect of precursors feeding and media manipulation on production of novel anticancer pro-drug camptothecin from endophytic fungus. Brazilian Journal of Microbiology, 43(4), 1476-1489. https://doi.org/10.1590/S1517-83822012000400032
4. Turk, A., Abdelhamid, M. A. A., Yeon, S. W., Ryu, S. H., Lee, S., Ko, S. M., Kim, B. S., Pack, S. P., Hwang, B. Y., & Lee, M. K. (2022). Cordyceps mushroom with increased cordycepin content by the cultivation on edible insects. Frontiers in Microbiology, 13, 1017576. https://doi.org/10.3389/fmicb.2022.1017576
5. Vesović, N., Nenadić, M., Vranić, S., Vujisić, L., Milinčic, K. M., Todosijević, M., Dimkić, I., Janakiev, T., Ćurčić, N. B., Stevanović, N., Mihajlović, L., Vukoičić, D. Ž., & Ćurčić, S. (2023). The chemical composition of the secretions, their antibacterial activity, and the pygidial gland morphology of selected European Carabini ground beetles (Coleoptera: Carabidae). Frontiers in Ecology and Evolution, 11, 1120006. https://doi.org/10.3389/fevo.2023.1120006
6. Yeo, H., Youn, K., Kim, M., Yun, E.-Y., Hwang, J.-S., Jeong, W.-S., & Jun, M. (2013). Fatty Acid Composition and Volatile Constituents of Protaetia brevitarsis Larvae. Preventive Nutrition and Food Science, 18(2), 150-156. https://doi.org/10.3746/pnf.2013.18.2.150
7. Li, X., Jiang, R., Wang, S., Li, C., Xu, Y., Li, S., Li, Q., & Wang, L. (2024). Prospects for cordycepin biosynthesis in microbial cell factories. Frontiers in Chemical Engineering, 6, 1446454. https://doi.org/10.3389/fceng.2024.1446454
8. Wang, L., Yan, H., Zeng, B., & Hu, Z. (2022). Research Progress on Cordycepin Synthesis and Methods for Enhancement of Cordycepin Production in Cordyceps militaris. Bioengineering, 9(2), 69. https://doi.org/10.3390/bioengineering9020069
9. Schenk, J. J., Becklund, L. E., Carey, S. J., & Fabre, P. P. (2023). What is the "modified" CTAB protocol? Characterizing modifications to the CTAB DNA extraction protocol. Applications in Plant Sciences, 11(3), e11517. https://doi.org/10.1002/aps3.11517
10. Mao, X.-B., & Zhong, J.-J. (2004). Hyperproduction of Cordycepin by Two-Stage Dissolved Oxygen Control in Submerged Cultivation of Medicinal Mushroom Cordyceps militaris in Bioreactors. Biotechnology Progress, 20(5), 1408-1413. https://doi.org/10.1021/bp049765r
11. Zhang, Q., & Liu, Y. (2016). The Strategies for Increasing Cordycepin Production of Cordyceps Militaris by Liquid Fermentation. Fungal Genomics & Biology, 6(1). https://doi.org/10.4172/2165-8056.1000134
12. Mao, X.-B., & Zhong, J.-J. (2004). Hyperproduction of Cordycepin by Two-Stage Dissolved Oxygen Control in Submerged Cultivation of Medicinal Mushroom Cordyceps militaris in Bioreactors. Biotechnology Progress, 20(5), 1408-1413. https://doi.org/10.1021/bp049765r

## Claims

1. A method for culturing *Cordyceps* fungi producing cordycepin, comprising the steps:
a) Obtaining a fungi inoculum,
b) Optionally, inoculating the fungi inoculum in a first liquid growth medium,
c) Inoculating fungi inoculum into a second liquid growth medium,
d) culturing under culturing conditions,
wherein:
(i) before or during step d) the second liquid growth medium is enriched with a source of oleic acid, and/or
(ii) culturing conditions include a pulsed hyperoxygenation phase, and/or
(iii) the *Cordyceps* species is a variant of *Cordyceps* species that has reduced capability to synthesize saturated fatty acids.

2. The method according to claim 1, wherein the *Cordyceps* species is selected from *Cordyceps militaris, Cordyceps sinensis (Ophiocordyceps sinensis), Cordyceps cardinalis, Cordyceps cicadae, Cordyceps kyushuensis* and *Cordyceps bassiana,* preferably wherein the *Cordyceps* species is *Cordyceps militaris.*

3. The method according to claim 1 or 2, wherein the fungi inoculum is obtained by inoculating the fungi mycelium or spores on a solid medium and incubating.

4. The method according to any one of claims 1-3, wherein the first liquid growth medium is the same as the second growth medium.

5. The method according to any preceding claim, wherein the source of oleic acid is insect-derived material.

6. The method according to claim 6, wherein the insect material is selected from the group consisting of insect biomass, insect biomass powder, insect extracts and insect homogenates.

7. The method according to claim 5 or 6, wherein the insect material is selected from insects comprising 5-65 % (w/w) oleic acid content of total fatty acids, preferably, 20-45% (w/w) oleic acid content.

8. The method according to any preceding claim, wherein the insect material is from insect family selected from the group consisting of *Tenebrionidae, Gryllidae, Acrididae, Acrididae, Bombycidae, Saturniidae, Formicidae, Scarabaeidae, Pyrrhocoridae, Carabidae, Lucanidae* insect families or combination thereof.

9. The method according to any preceding claim, wherein the insect material is from insect genus selected from *Tenebrio, Acheta, Schistocerca, Chorthippus, Bombyx, Cricula, Formica, Protaetia, Dysdercus, Antheraea, Calosoma, Dorcus,* or combination thereof.

10. The method according to any preceding claim, the insect material is from insect selected from *Tenebrio molitor, Acheta domesticus, Schistocerca gregaria, Chorthippus brunneus, Bombyx mori, Cricula trifenestrata, Formica rufa, Protaetia brevitarsis, Dysdercus koenigii, Antheraea pernyi, Calosoma scrutator, Dorcus hopei,* or combination thereof.

11. The method according to any preceding claim, wherein the variant of *Cordyceps* species that has reduced capability to synthesize saturated fatty acids is a Δfas1 mutant.

12. The method according to any preceding claim, wherein the pulsed hyperoxygenation phase comprises a step comprising at least four first hyperoxygenation cycles of varying dissolved oxygen levels, wherein each first hyperoxygenation cycle comprises:
(i) maintaining dissolved oxygen at 45%-55%, preferably at 50%, of air saturation for 4-5 hours, preferably for 4 hours, followed by
(ii) maintaining dissolved oxygen at 15%-20%, preferably at 20%, of air saturation for 2-3 hours, preferably for 2 hours.

13. The method according to claim 12, wherein the pulsed hyperoxygenation phase further comprises a step comprising at least four second hyperoxygenation cycles of varying dissolved oxygen levels, wherein each second hyperoxygenation cycle comprises:
(i) maintaining dissolved oxygen at 65%-75%, preferably at 70%, of air saturation for 4-5 hours, preferably for 4 hours, followed by
(ii) maintaining dissolved oxygen at 15%-20%, preferably at 20%, of air saturation for 2-3 hours, preferably for 2 hours.

14. The method according to claim 13, wherein the pulsed hyperoxygenation phase further comprises a step comprising at least four third hyperoxygenation cycles of varying dissolved oxygen levels, wherein each third hyperoxygenation cycle comprises:
(i) maintaining dissolved oxygen at 85%-95%, preferably at 90%, of air saturation for 4-5 hours, preferably for 4 hours, followed by
(ii) maintaining dissolved oxygen at 15%-20%, preferably at 20%, of air saturation for 2-3 hours, preferably for 2 hours.

15. The method according to any one of claims 12-14, wherein the second liquid growth medium is enriched with a source of oleic acid before or during the pulsed hyperoxygenation phase, preferably the second liquid growth medium is enriched with a source of oleic acid before or during a) the first cycle of the first hyperoxygenation cycles, b) the first cycle of the second hyperoxygenation cycles, and/or c) the first cycle of the third hyperoxygenation cycles.
